# EUROPEAN PATENT SPECIFICATION

(11) **EP 1 933 753 B1**
(45) Date of publication and mention of the grant of the patent: **19.01.2011**
(21) Application number: 06809417.6
(22) Date of filing: 27.09.2006
(51) Int. Cl.: A61B 18/20, B26B 19/44

(54) **A DEVICE FOR REMOVING HAIRS FROM SKIN**
VORRICHTUNG ZUR HAARENTFERNUNG AUF DER HAUT
DISPOSITIF D'EPILATION DE LA PEAU

(30) Priority: 03.10.2005 EP 05109141
(43) Date of publication of application: 25.06.2008
(73) Proprietor: Koninklijke Philips Electronics N.V., 5621 BA Eindhoven (NL)
(72) Inventor: VAN HAL, Robbert, A., M., NL-5656 AA Eindhoven (NL); ZUIDERVAART, Jasper, NL-5656 AA Eindhoven (NL); HOUBOLT, Erik, NL-5656 AA Eindhoven (NL); NIEHAUS, Mathijs, NL-5656 AA Eindhoven (NL); VERHAGEN, Rieko, NL-5656 AA Eindhoven (NL); ACKERMANS, Paul, A., J., NL-5656 AA Eindhoven (NL); NUIJS, Antonius, M., NL-5656 AA Eindhoven (NL)
(74) Representative: Uittenbogaard, Frank
(86) International application number: PCT/IB2006/053510
(87) International publication number: WO 2007/039853

(56) References cited:
- WO-A-2005/011510
- GB-A- 1 292 351
- US-A1- 2 249 293
- US-A1- 5 606 798
- US-A1- 2001 027 608

## Description

The invention relates to a device for removing hairs from skin, comprising an optical device for shortening hairs, that is constructed and arranged for delivering optical energy to a hair, such that the hair will be severed from a remaining hair part, and a debris removal system, that is constructed and arranged for removing the severed hair from the skin.

Devices for removing hair from skin have been known since ancient times. In recent times, optical devices for shaving etc. have been developed. For all devices, it is desirable that the severed hair and/or other debris are removed from the skin after shaving, epilating etc. Otherwise, the severed hairs and debris would still be noticeable on the skin, or would be diffused over the skin, such as the face, and over clothes etc. of the user.

For example, US 5,606,798 discloses a hair cutting apparatus with a hair collection apparatus. The hair cutting apparatus includes a housing and a laser apparatus in the housing. Cut hairs are collected inside the housing by a vacuum device or electrostatic hair collection apparatus.

A problem of the known device relates to the following. Since the optical cutting of hairs is a contactless way of cutting hairs, said devices and the methods they employ cannot remove cut hairs by themselves. The known device also uses contactless devices or methods in trying to remove the cut hairs. However, such contactless methods, *viz*. vacuum suction or electrostatic attraction, are not always able to remove the cut hairs in a reliable fashion, since the forces that may be exerted on the cut hair are rather limited. This holds in particular for cut hairs that stick to the skin, in particular to e.g. fatty substances, water on the skin, etc.

It is an object of the invention to provide a device of the type mentioned above, that is able to remove hairs from skin in a more reliable way.

This object is achieved with a device of the type mentioned above, that is **characterized in that** the debris removal system comprises a remover part that is constructed and arranged to mechanically engage the severed hair to remove the severed hair from the skin. By providing a remover part that is constructed and arranged to mechanically engage the severed hair to remove the severed hair from the skin, it is ensured that a much higher force may be exerted on the severed hair. This is due to the fact that, by mechanically engaging the hair, mechanical contact between the hair and the device, in particular the remover part, is established.

In particular, the contact between the hair and the remover part relates to establishing a frictional force between the surface of the severed hair and a surface of the remover part. It is noted that adhesion between a surface of the severed hair and a surface of the remover part is deemed to be comprised in the expression "frictional force". A true frictional force between a severed hair and a solid surface is a special embodiment thereof.

In a special embodiment, the optical device is constructed and arranged for severing the hair subcutaneously, preferably at least 0.1 mm and more preferably at least 0.35 mm below the skin surface. This embodiment, which allows hair removal for subcutaneous optical hair shortening, is particularly efficient for the following reasons. First of all, it is noted that the expression "subcutaneous" is meant to intend "taking place below the surface of the skin". Shortening hairs below the surface of the skin is desirable since it will take longer for the growing hairs to protrude from the skin. Since an average beard hair grows about 0.35 mm per day, it is possible to maintain a smooth skin all day long by optical shaving once a day at a depth of 0.35 mm or more below the surface of the skin. Practical embodiments of such optical devices are known in the art, for example from WO 00/62700 and WO 2005//011510. Further details may be found in these documents. Now, when shortening a hair below the surface of the skin, the severed hair will at least partly be present inside the hair shaft. This severed portion will then stick in the shaft, due to mechanical effects such as friction with the wall of the shaft, possibly even more so due to fatty substances such as sebum etc. This means that the known methods and devices will not, or at least not reliably, be able to remove those severed hair portions from the shaft. This is even more so if the severed hair portions are very short, in particular if the hairs are regularly shortened such as in shaving. The device of the present embodiment is then particularly suited to remove the severed hairs, since by mechanically engaging the severed hair, it is much easier to exert a sufficiently large force on the hair.

It is noted that the severed hair is no longer physiologically connected to the skin when the hair is severed up to a depth of around the sebaceous duct, since the sebum that is excreted by the sebaceous gland will dissolve the inner root sheath. Below the sebaceous duct, the hair is more or less connected to the skin by said inner root sheath. The force required to remove a hair that is severed below said sebaceous duct increases rather sharply as compared to the force required to remove a hair that is severed up to said sebaceous duct. Said force will become much higher with increasing depth and thus cause a sensation of pain, e.g. comparable with epilating by hand. Hence, a preferred depth of severing the hairs is up to the level of the sebaceous duct. This depth varies between about 0.25 and 1 mm.

In a special embodiment, the remover part is constructed to be moved across and in contact with the skin, during use of the device on skin. Such a remover part ensures a good mechanical engagement with the hairs, since the distance to the hairs will be minimal.

In a particular embodiment, the remover part comprises a scraper that, during operation of the device, scrapes across the skin to engage the severed hair. The scraper enables a good mechanical contact with the severed hairs. It may be adapted to increase the mechanical engagement forces, such as frictional forces. For this purpose, the material of the scraper may be selected on the basis of the coefficient of friction with hair material, for example in particular rubbery materials. It is also possible to adapt the shape of the scraper. For example, the scraper may comprise a single ridge that is moved across the skin surface. It may, however, comprise a number of adjacent ridges in order to increase the action on the skin and to increase the chances of a severed hair actually being removed by the scraper.

In use of the device, the scraper may not only pull the severed hair from the shaft through mechanical engagement, it may also be able to push or topple the hair, in particular if the depth at which the hair is severed is relatively small, such as a few tenths of a millimeter.

In a special embodiment, the remover part comprises a moveable part, that is constructed to move with respect to the skin, independent of a movement of the device as a whole. By making the remover part moveable independently of the device as a whole, said movability may be optimized. Note that "moveable" comprises actively (=driven by a motor) moveable as well as passively moveable, such as by friction with the skin or hair. As an example, a dedicated remover part motor may actively move the remover part across the skin in for example a vibratory fashion. A frequency and amplitude of such a vibration may be optimized to achieve an optimum result.

In another special embodiment, the moveable part is rotatable around an axis, preferably an axis that extends substantially parallel to the skin, in use of the device. This embodiment with a part that is rotated around an axis offers the advantage that it is very easy to provide such a motion by a very simple motor, or even by other external means, such as friction or an air/fluid current. It is possible to obtain a relatively high velocity of the remover part, without the accompanying vibrations for most non-rotary motions.

In a particular embodiment, the remover part comprises a brush. The brush may also be moveable and/or rotatable around an axis. The brush may comprise a plurality of hairs for engaging the severed hairs. By choosing an appropriate number of hairs, in particular a large number thereof, it is ensured that the severed hairs may be reliably removed from the skin, even when the severed hairs are only short in length.

In another special embodiment, the debris removal system comprises a holder for an adhesive for application to the skin. The mechanical engagement may also be brought about by applying adhesive to the skin. As mentioned above, the mechanical force is then brought about by the adhesion between the adhesive and the severed hairs. In the present embodiment, the device is adapted thereto in that it comprises a holder for such an adhesive. Herein, a holder may comprise any part on the device that is constructed and designed to hold an adhesive.

In a particular embodiment, the holder comprises an adhesive tape. An example of an adhesive tape for use in this device is a depilatory prewaxed tape. Alternatively, any other adhesive tape that has an appropriate adhesion to severed hairs, and an adhesion to skin that will not cause excessive pain when removing the strip, is suitable. In the case of the present embodiment, the holder for the adhesive could comprise for example a reel.

In another special embodiment, the remover part comprises adhesive fluid, that expediently exerts sufficient adhesive force on the severed hairs for it to carry off those severed hairs when the adhesive fluid is being removed. The adhesive fluid is removed, for example, by rinsing or peeling it off the skin.

In a special embodiment, the remover part comprises a container for adhesive fluid, and a dispensing opening for dispensing the fluid onto the skin. In use, the user may dispense fluid onto the skin, e.g. after shortening the hairs. The severed hair may then be removed by removing the fluid from the skin, by rinsing, scraping, etc.

In a special embodiment, the debris removal system in addition comprises a suction device constructed to create a pressure lower than ambient pressure on a part of the skin with severed hair to be removed. Such a suction device may assist in removing severed hair or debris, or products such as adhesives that engage or adhere to the severed hair. Even when the suction device cannot remove severed hair by itself, it may assist in removing severed hair by increasing the total force exerted on the severed hair. Furthermore, such a suction device may also assist in preventing spreading of the severed hair or debris by positively determining a direction into which the severed hair or debris is carried off.

In a special embodiment, the suction device comprises at least one of an air suction device and a fluid suction device. An air suction device may for example be a vacuum suction device, which in itself is well known in the art. Such a device may assist in collecting hairs that happen to be already loosening from the skin. Otherwise one would run the risk that these hairs spread across the skin, clothes etc. before being engaged by the remover part. Furthermore, if removed severed hairs happen to get loose from the remover part, the suction device, such as the air suction device, again assists in preventing spreading of the severed hairs across the skin etc.

A fluid suction device may assist in those cases where fluid is present on the skin. By sucking the fluid from the skin, any severed hairs that are present in (adhere to) the fluid may then also be sucked from the skin.

In a particular embodiment, the device according to the present invention comprises a container for collecting severed hair. Such a container serves to positively collect the severed hair in a controlled way. This prevents the severed hair from spreading in the device, where it may cause a malfunction or non-hygienic circumstances etc. Advantageously, the container is removable, which allows a user of the device to empty the container for subsequent use. In another advantageous embodiment, the container comprises a filter, through which air and/or fluid may pass but which substantially filters out severed hair. For example, and in particular in the case of suction devices, the positive direction of the air flow or fluid flow serves to collect the severed hair in a more controlled fashion by leading the air flow or fluid flow through the filter.

In a special embodiment, the device comprises a pressure exertion part that, during use of the device on skin, compresses the skin such that at least either the remaining hair part or the severed hair are displaced in a direction out of the skin. Such a pressure exertion part may be advantageous in the following way. By compressing the skin, various outer skin layers become locally thinner. However, the less compressible hair, both the part that remains in the skin and the severed part, will not show a similar decrease in length. This means that those parts of the hair will move relative to those skin layers, *viz*. the outer skin layers. The effect thereof is that those parts are more accessible from the outside. This may serve to more easily shorten the hair at a certain depth below the skin in uncompressed condition. But, in particular, it also serves to move the severed hair part upward in the hair shaft to thereby decrease the force with which the severed hair part might stick in said hair shaft. This accordingly decreases the force required to remove the severed hair from the hair shaft. One could say that the remaining hair part serves as an "anvil" for the severed hair part in pushing it outward.

In a particular embodiment, the pressure exertion part comprises a ridge that at least partly surrounds the remover part. Such a ridge, or rim or other protrusion, can effectively compress the outer skin layers locally. It need not do so all around the removal part, although it is a further advantageous embodiment.

These and other aspects of the invention are apparent from and will be elucidated with reference to the embodiments described hereinafter.

In the drawings:
Fig. 1 diagrammatically shows an embodiment of device of the present invention;
Figs. 2a -2c show details of other embodiments of devices according to the present invention.

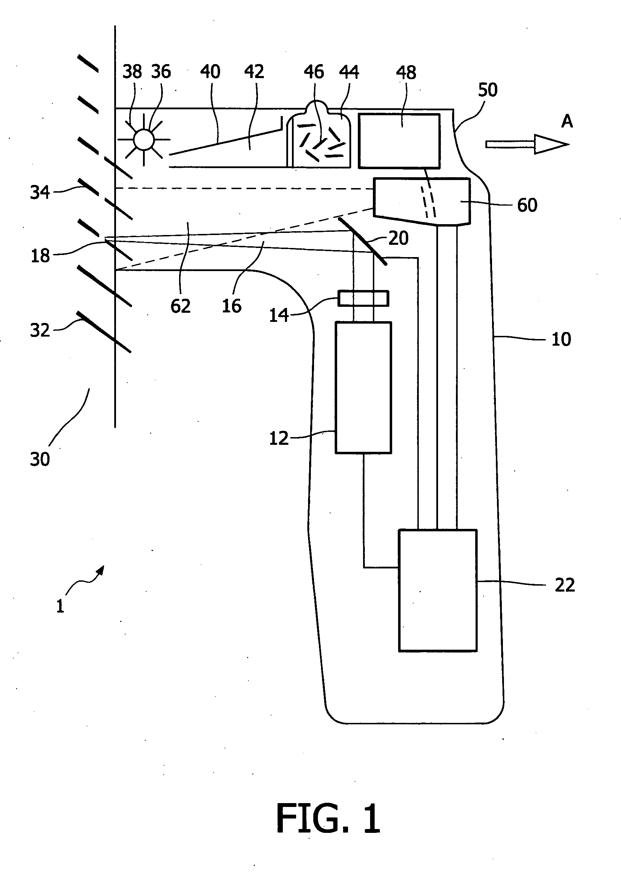

Figure 1 diagrammatically shows a device 1 according to the present invention. The device 1 comprises a housing 10 with a laser source 12 which, through optical system 14, emits a laser beam 16 with a focus 18. An optical beam manipulator is designated with reference numeral 20, while a control unit is denoted with 22.

Skin 30 has hairs 32 and remaining hair parts 34.

A brush 36 with hairs 38 is located in front of a suction housing 40 with a suction duct 42, that is connected to a container 44 filled with severed hairs 46. 48 denotes a suction device with an exit opening 50 in the housing. 60 denotes an imaging device with a field-of-view 62.

The housing 10 may have any appropriate shape and may be made of any appropriate material, such as metal or plastics.

The laser source 12 may be a suitable laser source for shortening hair, such as disclosed in e.g. WO 00/62700. In particular, the laser source is arranged to generate a LIOB phenomenon in the hairs 32, such that the hairs 32 may be cut beneath the surface of the skin 30. Note, however, that such a LIOB generating laser source 12 is not the only type of laser source to cause subcutaneous cutting of the hairs 32. Furthermore, it is not excluded to use other types of optical sources, such as pulsed LEDs.

The optical system 14 focuses the emitted laser beam 16 into a focus 18. Such a focus 18 may be present above, on or below the surface of the skin 30. In the focus, the power density and energy is such that the hairs 32 will be cut. After cutting, there will be a remaining hair part 34, as well as a severed hair 46.

The laser beam is directed towards the skin 30 via a laser beam manipulator 20. The laser beam manipulator 20 may for example be a movable, such as pivotable, mirror. The laser beam manipulator 20 is controlled by means of a control unit 22, that may also be connected to the laser source 12 as well as to a hair imaging device 60. The hair imaging device 60 may comprise e.g. a CCD camera. The hair imaging device has a field-of-view 62. By forming images of the hairs 32 of the skin 30, the imaging system 60 is able to determine a target position for the focus 18. The control unit 22 accordingly directs the focus 18 towards the target position by moving the beam manipulator 20.

After shortening, or severing, the hairs 32, the severed hairs 46 will be present on or in the skin. To remove the severed hairs 46 reliably, the device 1 comprises a mechanical remover part, in this case a brush 36 with hairs 38. The brush 36 may rotate around its axis. The hairs 38 will mechanically engage the severed hairs 46, and remove them from the surface of the skin 30. To assist in removing the severed hairs 46 even more reliably, there is optionally provided a suction device, comprising a suction housing 40 with a suction duct 42 through which air may be sucked by a suction device 48. This air may exit the housing 10 via exit opening 50 in the direction of arrow A. The severed hairs 46 will be entrained by the flow of air and collected in the container 44. The container 44 may be provided with a filter (not shown), such that air may pass and the severed hair 46 will remain in the container 44. Preferably, the container 44 is removable, such that it may be emptied and/or cleaned.

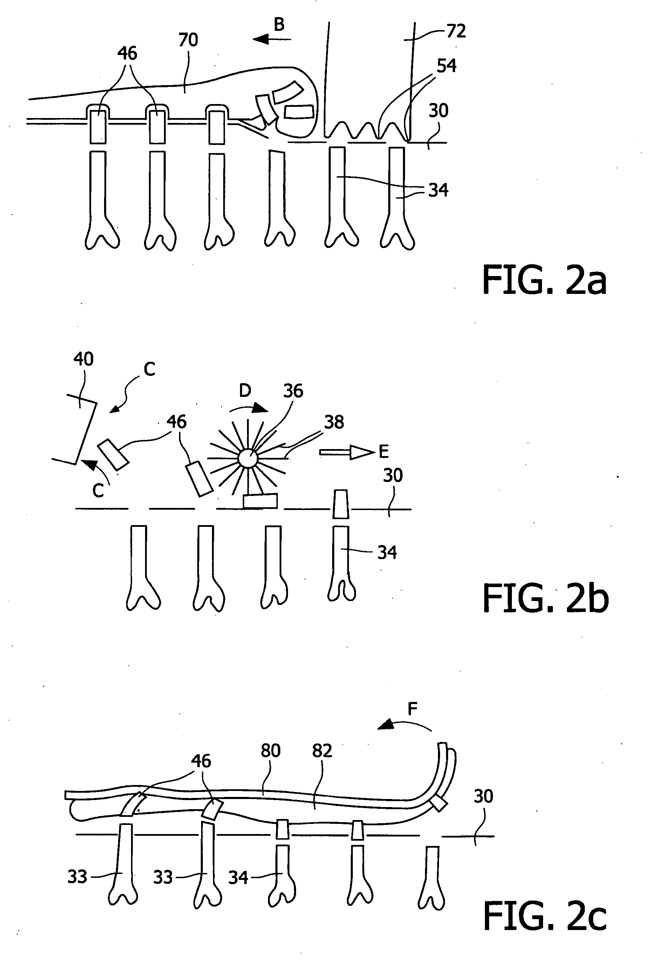

Figure 2a shows a detail of another embodiment of the present invention. There is shown an adhesive 70, contacting severed hair 46 on skin 30. Furthermore, 72 denotes a pressure exertion part which is also a scraper, and which has ridges 74.

In the embodiment shown, hairs 32 have been severed below the surface of the skin 30, such that severed hairs 46 remain sticking in the hair shaft. To remove the severed hairs 46, an adhesive 70, such as a suitable emulsion, wax, etc., has been applied to the skin 30. The device according to the invention may comprise a container for the adhesive 70, with a dispensing opening, not shown.

It is possible to remove the adhesive 70 together with the severed hairs 46 by e.g. rinsing, or wiping with a towel. In order to assist in removing the adhesive, use may be made of the scraper 72, which is moved across the skin in the direction of arrow B. The scraper will scrape the adhesive 70, together with the severed hairs 46, off the surface of the skin 30. To improve the contact between the scraper 72 and the skin 30, the scraper 72 may comprise ridges 74 that ensure that optimum mechanical engagement with the severed hairs 46 will be established.

As mentioned above, the scraper 72 may also serve as a pressure exertion part. In use of the device according to the invention, the pressure exertion part 72 will be pushed against the skin 30. Then, as is shown in Figure 2a, the upper layers of the skin 30 will be compressed, while the remaining hair parts 34 are substantially not displaced. The nett effect of this is that the severed hairs 46 will be at least partially pushed out of the hair shaft. Hence, the force with which they stick in the hair shaft, and hence the force required to remove the severed hairs 46 from the skin, will be reduced.

Note that in the above, both the adhesive 70, and the scraper 72 and/or the pressure exertion part 72, may be used in combination or separately. In particular, although the part 72 has a double function in this Figure, they may be separate parts.

Figure 2b shows another embodiment of a detail of the device according to the present invention. Herein, as in all Figures, similar parts are denoted by the same reference numerals. In particular, 40 is a part of a suction housing, a brush 36 with hairs 38 is rotatable in the direction of arrow D and engages severed hairs 46, which may be sucked away in the direction of arrows C. The device as a whole, comprising inter alia the brush 36 and the suction housing 40, may be moved across the skin 30 in the direction of arrow E.

This embodiment more or less corresponds to the detail of the device 1 shown in Figure 1. In use of this device, the hairs 38 of the brush 36 will mechanically engage the severed hairs 46 and remove them from the skin 30. To assist in unambiguously removing the severed hairs 46, air, or possibly some kind of fluid, may be sucked away from the skin 30, in this case in the direction of arrows C. The severed hairs 46 will be entrained in the airflow and may be removed via the suction housing 40. By moving the device in the direction of arrow E, the brush 36 may be applied to all of the desired skin surface.

Figure 2c shows another embodiment of a detail of the device according to the present invention. Herein, 80 denotes a tape with a layer of adhesive material 82.

The tape is applied to the skin 30 that comprises remaining hairs 34 and second remaining hairs 33. Note that the hairs of which 34 is the remaining hair have been severed below the surface of the skin 30, while the second remaining hairs 33 belong to hairs that have been severed above the surface of the skin 30. This is to indicate that the present invention is not limited to removal of severed hairs that have been severed below the surface of the skin, although it has advantages for this application.

In use of this embodiment, the tape 80 may be applied to the surface of the skin 30 by unwinding a reel of the tape 80 in contact with the skin 30. The adhesive material 82 on the tape 80 will contact the severed hairs 46. By subsequently peeling off the tape 80, e.g. in the direction of the arrow F, the tape including the adhesive material 82 will remove the severed hairs 46 from the skin 30. If need be, the adhesive material 82 will pull the severed hairs 46 out of the hair shaft, in case they should stick to the hair shaft. Of course, in the case of the second remaining hairs, the adhesive material will simply remove the severed hairs 46, and other debris, from the skin.

## Claims

1. A device for removing hairs (32) from skin (30), comprising
- an optical device for shortening hairs, that is constructed and arranged for delivering optical energy to a hair (32), such that the hair will be severed from a remaining hair part (34), and
- a debris removal system, that is constructed and arranged for removing the severed hair (46) from the skin (30),
**characterized in that** the debris removal system comprises a remover part (36; 72; 80) that is constructed and arranged to mechanically engage the severed hair (46) to remove the severed hair from the skin (30).

2. A device according to claim 1, wherein the optical device is constructed and arranged for severing the hair (32) subcutaneously, preferably at least 0.1 mm and more preferably at least 0.35 mm below the surface of the skin (30), by delivering the optical energy to one or more subcutaneous points in or on the hair (32).

3. A device according to claim 1, wherein the remover part (36; 72; 80) is constructed to be moved across and in contact with the skin (30), during use of the device (1) on skin.

4. A device according to claim 1, wherein the remover part comprises a scraper (72) adapted to scrape across the skin (30) to engage the severed hair (46).

5. A device according to claim 1, wherein the remover part comprises a moveable part (36; 80) that is constructed to move with respect to the skin (30), independent of a movement of the device (1) as a whole.

6. A device according to claim 5, wherein the moveable part (36) is rotatable around an axis, preferably an axis that extends substantially parallel to the skin (30), in use of the device (1).

7. A device according to claim 1, wherein the remover part comprises a brush (36).

8. A device according to claim 1, wherein the debris removal system comprises a holder for an adhesive (70; 80, 82) for application to the skin (30).

9. A device according to claim 8, wherein the holder comprises an adhesive tape (80, 82).

10. A device according to claim 8 or 9, wherein the remover part comprises a container for adhesive fluid (70) and a dispensing opening for dispensing the fluid onto the skin (30).

11. A device according to claim 1, wherein the debris removal system additionally comprises a suction device (48) constructed to create a pressure lower than ambient pressure on a part of the skin (30) with severed hair (46) to be removed.

12. A device according to claim 11, wherein the suction device comprises at least one of an air suction device (48) and a fluid suction device.

13. A device according to claim 1, comprising a container (44) for collecting severed hair (46).

14. A device according to claim 1, comprising a pressure exertion part (72) adapted to, during use of the device (1) on skin (30), compress the skin such that at least one of the remaining hair part (34) and the severed hair (46) are displaced in a direction out of the skin (30).

15. A device according to claim 14, wherein the pressure exertion part comprises a ridge that at least partly surrounds the remover part.

## Patentansprüche

1. Vorrichtung zum Entfernen von Haaren (32) auf der Haut (30), mit
- einer optischen Vorrichtung zum Kürzen von Haaren, die so ausgeführt und eingerichtet ist, dass sie einem Haar (32) optische Energie zuführt, so dass das Haar von einem verbleibenden Haarteil (34) abgetrennt wird, sowie
- einem System zum Entfernen der Rückstände, das so ausgeführt und eingerichtet ist, dass es das von der Haut (30) abgetrennte Haar (46) entfernt,
**dadurch gekennzeichnet, dass** das System zum Entfernen der Rückstände einen Entfernerteil (36; 72; 80) umfasst, der so ausgeführt und eingerichtet ist, dass er mechanisch in das abgetrennte Haar (46) eingreift, um das abgetrennte Haar von der Haut (30) zu entfernen.

2. Vorrichtung nach Anspruch 1, wobei die optische Vorrichtung so ausgeführt und eingerichtet ist, dass sie das Haar (32), indem sie einem oder mehreren subkutanen Punkten in oder auf dem Haar (32) die optische Energie zuführt, subkutan, vorzugsweise mindestens 0,1 mm, noch besser mindestens 0,35 mm, unter der Oberfläche der Haut (30) abtrennt.

3. Vorrichtung nach Anspruch 1, wobei der Entfernerteil (36; 72; 80) so ausgeführt ist, dass er während der Benutzung der Vorrichtung (1) auf der Haut über die Haut (30) und in Kontakt mit dieser bewegt wird.

4. Vorrichtung nach Anspruch 1, wobei der Entfernerteil einen Schaber (72) umfasst, der so ausgebildet ist, dass er über die Haut (30) gezogen wird, um in das abgetrennte Haar (46) einzugreifen.

5. Vorrichtung nach Anspruch 1, wobei der Entfernerteil einen bewegbaren Teil (36; 80) umfasst, der so ausgebildet ist, dass er sich, unabhängig von einer Bewegung der Vorrichtung (1) als Ganzes, gegenüber der Haut (30) bewegt.

6. Vorrichtung nach Anspruch 5, wobei der bewegbare Teil (36) bei Gebrauch der Vorrichtung (1) um eine Achse, vorzugsweise um eine Achse, die sich im Wesentlichen parallel zu der Haut (30) erstreckt, drehbar ist.

7. Vorrichtung nach Anspruch 1, wobei der Entfernerteil eine Bürste (36) umfasst.

8. Vorrichtung nach Anspruch 1, wobei das System zum Entfernen der Rückstände einen Halter für ein Haftmittel (70; 80, 82) zum Aufbringen auf die Haut (30) umfasst.

9. Vorrichtung nach Anspruch 8, wobei der Halter ein Haftklebeband (80, 82) umfasst.

10. Vorrichtung nach Anspruch 8 oder 9, wobei der Entfernerteil einen Behälter für Haftfluid (70) sowie eine Abgabeöffnung zum Abgeben des Fluids auf die Haut (30) umfasst.

11. Vorrichtung nach Anspruch 1, wobei das System zum Entfernen der Rückstände zudem eine Saugvorrichtung (48) umfasst, die so ausgeführt ist, dass sie auf einem Teil der Haut (30) mit zu entfernenden, abgetrennten Haaren (46) einen niedrigeren Druck als den Umgebungsdruck erzeugt.

12. Vorrichtung nach Anspruch 11, wobei die Saugvorrichtung zumindest eine Luftansaugvorrichtung (48) oder eine Fluidansaugvorrichtung umfasst.

13. Vorrichtung nach Anspruch 1, mit einem Behälter (44) zum Sammeln abgetrennter Haare (46).

14. Vorrichtung nach Anspruch 1, mit einem Druckbeaufschlagungsteil (72), der so eingerichtet ist, dass er bei Verwenden der Vorrichtung (1) auf der Haut (30) die Haut so zusammendrückt, dass zumindest der verbleibende Haarteil (34) oder das abgetrennte Haar (46) in Richtung aus der Haut (30) heraus verschoben wird.

15. Vorrichtung nach Anspruch 14, wobei der Druckbeaufschlagungsteil eine Rille umfasst, die zumindest zum Teil den Entfernerteil umgibt.

## Revendications

1. Dispositif d'élimination de poils (32) de la peau (30), comprenant :
- un dispositif optique pour raccourcir les poils, construit et agencé pour délivrer de l'énergie optique à un poil (32), de telle manière que le poil soit coupé d'une partie de poil (34) restante, et
- un système d'élimination de déchets construit et agencé pour éliminer le poil coupé (46) de la peau (30),
**caractérisé en ce que** le système d'élimination de déchets comprend une partie de dispositif d'élimination (36 ; 72 ; 80) construite et agencée pour prendre mécaniquement le poil coupé (46) pour éliminer le poil coupé de la peau (30).

2. Dispositif selon la revendication 1, dans lequel le dispositif optique est construit et agencé pour couper le poil (32) de façon sous-cutanée, de préférence au moins 0,1 mm et plus préférablement au moins 0,35 mm en dessous de la surface de la peau (30), en délivrant l'énergie optique à un ou plusieurs points sous-cutanés dans ou sur le poil (32).

3. Dispositif selon la revendication 1, dans lequel le dispositif d'élimination (36 ; 72 ; 80) est construit pour être déplacé en travers et en contact avec la peau (30), pendant l'utilisation du dispositif (1) sur la peau.

4. Dispositif selon la revendication 1, dans lequel la partie de dispositif d'élimination comprend un grattoir (72) adapté pour gratter en travers de la peau (30) pour prendre le poil coupé (46).

5. Dispositif selon la revendication 1, dans lequel la partie de dispositif d'élimination comprend une partie mobile (36 ; 80) construite pour se déplacer par rapport à la peau (30), indépendamment d'un mouvement du dispositif (1) dans son ensemble.

6. Dispositif selon la revendication 5, dans lequel la partie mobile (36) est rotative autour d'un axe, de préférence un axe s'étendant sensiblement parallèlement à la peau (30), pendant l'utilisation du dispositif (1).

7. Dispositif selon la revendication 1, dans lequel la partie du dispositif d'élimination comprend une brosse (36).

8. Dispositif selon la revendication 1, dans lequel le système d'élimination de déchets comprend un support pour un adhésif (70 ; 80, 82) pour l'application sur la peau (30).

9. Dispositif selon la revendication 8, dans lequel le support comprend une bande adhésive (80, 82).

10. Dispositif selon la revendication 8 ou 9, dans lequel la partie de dispositif d'élimination comprend un récipient de fluide adhésif (70) et une ouverture de distribution pour distribuer le fluide sur la peau (30).

11. Dispositif selon la revendication 1, dans lequel le système d'élimination de déchets comprend également un dispositif d'aspiration (48) construit pour créer une pression inférieure à la pression ambiante sur une partie de la peau (30) ayant le poil coupé (46) devant être éliminé.

12. Dispositif selon la revendication 11, dans lequel le dispositif d'aspiration comprend au moins l'un d'un dispositif d'aspiration d'air (48) et d'un dispositif d'aspiration de fluide.

13. Dispositif selon la revendication 1, comprenant un récipient (44) pour recueillir le poil coupé (46).

14. Dispositif selon la revendication 1, comprenant une partie d'application de pression (72) adaptée pour comprimer la peau pendant l'utilisation du dispositif (1) sur la peau (30), de telle manière qu'au moins l'une d'une partie du poil restante (34) et du poil coupé (46) soit déplacée dans une direction hors de la peau (30).

15. Dispositif selon la revendication 14, dans lequel la partie d'application de pression comprend une arête qui entoure au moins partiellement la partie du dispositif d'élimination.
